# EUROPEAN PATENT APPLICATION

(11) **EP 1 779 855 A1**
(43) Date of publication of application: **02.05.2007**
(21) Application number: 05077473.6
(22) Date of filing: 28.10.2005
(51) Int. Cl.: A61K 33/00, A61K 9/14, C01B 13/14

(54) **Nanoparticles of a heterocrystal mineral for use as a medicament and method of producing the same**

(71) Applicant: Kurkayev, Abdula, Angyal Utca 13 apt. 2 1094 Budapest (HU)
(72) Inventor: Kurkayev, Abdula, Angyal Utca 13 apt. 2 1094 Budapest (HU)
(74) Representative: Winckels, Johannes Hubertus F.

(57) **Abstract**

The invention relates to nanoparticles obtained by destruction of minerals for use as a medicament. Such nanoparticles exhibit high intrinsic activity, anti-bacterial, anti-viral action and can also be used for treatment of cancer and for healing wounds. Preferably, rutiles, sphenes, loparites, perowskites, anatases, ilmenites, leukoxenes, ferrites and quartzites are used for production of nanoparticles. Preferably a layer 12 of a mineral source is subjected to a thermal destruction by application of alternating cooling and heating modes, preferably using a thermo-element.

## Description

The invention relates to nanoparticles, in particular to nanoparticles for use in medicine.

An embodiment of a substance for a therapeutic use, based on its properties for generating chemically active reagents, is known from WO 96/26730. The known material comprises an organometallic compound, which is used for generating active chemical species at mildly acidic pH. The active chemical species act on cells causing their damage due to generation of active radicals and active oxygen in course of chemical reactions.

It is a disadvantage of the known substance that its chemical activity is highly dependent on the degree of acidity of the tissue, which has to be artificially kept at a certain level, by means of, for example, an application of hypo- or hyperthermia.

It is an object of the invention to provide a substance for use as a medicament which exhibits high intrinsic chemical and biologic activity.

To this end, according to the invention nanoparticles of a heterocrystal mineral are provided for use as a medicament. Within the terms of current invention, nanoparticles in particular refer to dimensions of 0,5 - 200 nm, more preferably to dimensions of 0,5 - 100 nm, more preferably to dimensions of 0,5 - 50 nm. The nanoparticles may have spheric and/or rod shapes, their dimensions being measured by per se known Atomic Force Microscope (AFH) or Stonnich Tunnel (ST). It is noted that nanoparticles may comprise various mixtures of nanoparticle sizes, whereby also relative percentage of nanoparticles of specific dimensions may vary. The term dimension relates to all dimensions of nanoparticles. By the term heterocrystal mineral one understands a substantially chemically homogenous substance, developed in a crystal within different lattices with respect to their types and shapes yielding polymorphic material, which can be detected by a per se known DSC method.

In the art it is understood that nanoparticles have dimensions comparable with dimensions of glucose molecule, and are thereby thousands of times smaller than an average size of a human cell. Therefore, nanoparticles can participate in chemical and biological reactions in a comparable way as molecules of matter, their properties being dependent on the properties of materials they are prepared from. An embodiment of a mineral (graphite) reduced to nanoparticles is known from RU 1263840. The known material is used as a filter for purifying water.

The technical measure of the invention is based on the insight that nanoparticles meet requirements for their application in medicine due to the fact that their high chemical activity is an intrinsic property and substantially no additional measures have to be taken to increase their specific chemical or biological activity in a recipient, it being a human or animal. The invention is based on a further insight that nanoparticles have a property of desorbing many earlier absorbed materials in aqueous medium, which is particularly useful for medical purposes. Nanoparticles of Si02 are of particular interest with his respect. Nanoparticles as such, notably comprising a bio-compatible composition for use as a medicament are contemplated as well. Preferably, minerals with a heterocrystal structure are used to provide nanoparticles, notably rutile (Ti02; (Ti, Nb, Fe)02), sphene (CaO.SiO2,TiO2), loparite (Ti,Nb)2(Na, Ca, Ce)206), perowskite (CaTi03), anatase (Ti02, (Ti, Nb, Fe)02), ilmenite (Fe1.10Ti90O3; FeTi03; Fe+2TiO3; (Fe, Mg)(Ti, Fe)03, Fe+2TiO3, (Fe, Mg)Ti03; Fe+2TiO3/Fe+20.TiO2)), leukoxen (TiO2Fe2O3+nH2O/Cr, Al, Si, P, Nb, Zr, Tr, Ta) ferrite (Fe203, Fe02), quartzite (Si02). These minerals are abundant in nature and can easily be destructed to nanoparticle size. Therefore, by selecting these minerals for production of nanoparticles for use as a medicament an efficient and relatively cheap source of useful medicaments is provided. By the term heterocrystal mineral one understands a substantially chemically homogenous substance, developed in a crystal within different lattices with respect to their types and shapes yielding polymorphic material which can be detected by a per se known DSC method.

Ferrites belong to a class of materials comprising dioxide of iron, most of them being ferromagnetic. Ferrites can refer to either a natural mineral or a chemically produced material using Fe203. Rutile is a natural mineral being a major ore of titanium, a metal used for high tech alloys because of its light weight, high strength and resistance to corrosion. Microscopic inclusions of rutile can be found in quartz, tourmaline, ruby, sapphire. Rutilated quartz can also be selected as a mineral source for production of nanoparticles. This stone is produced because at high temperatures and pressure, n(SiO2)-n(TiO2) is in a stable state but as temperatures cool and pressure eases the two separate with rutile crystals trapped inside the quartz crystals. Other suitable heterocrystal minerals being sources of nanoparticles, notably comprising TiO2 comprise sphene, loparite, perowskite, anatase, ilmenite, leukoxen. Quartzite is a natural mineral composed almost entirely of quartz grains, derived from sandstone or from chert. Sandstones turn into quartzite in two different ways. The first way involves low pressure and temperature, where circulating fluids fill the spaces between sand grains with silica cement. When this rock is broken, the fractures go right through the original grains, not around them. This kind of quartzite, orthoquartzite, is not strictly speaking a metamorphic rock because the original mineral grains are still there, and bedding planes and other sedimentary structures are still evident. Under the high pressures and temperatures of deep burial, the mineral grains recrystallize and all traces of the original sediments are erased. The result is a true metamorphic rock, called metaquartzite.

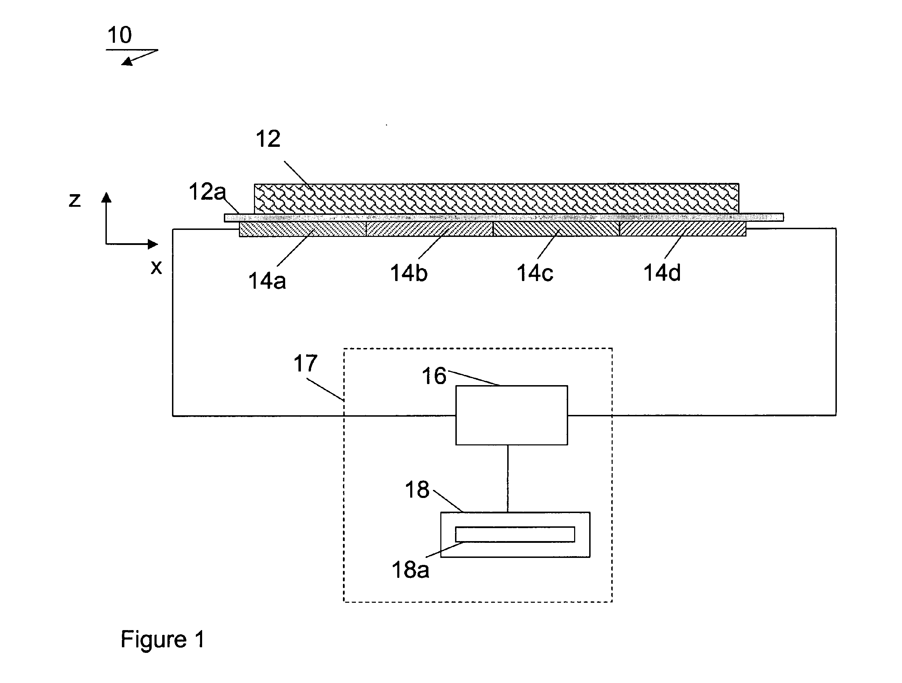

The technical measure of the invention is based on the insight that when a heterocrystal mineral is destructed to yield nanoparticles, the resulting nanoparticles comprise fragments of lattice with unsaturated bonds thereby providing particles with increased chemical activity, as active groups capable of initiating covalent and ionic bonds are notably formed at parts of intermolecular breaks. The technical measure of the invention is based on a further insight that practically all toxins, as well as inducers or mediators of pathologic processes in an organism belong to aggressive chemical compounds, having an elevated reaction potential for participating in chemical reactions of various kinds. Energetic neutralization of free chemical bonds of such toxic materials due to their saturation by means of complementary nanoparticles can stop predominantly all exogenic and edogenic intoxications. In case when the nanoparticles themselves doe not manifest any toxic effect, and can easily be excreted out of the organism, such class of materials is particularly advantageous for use as detoxifying agents. Therefore, nanoparticles can be used as a medicament as such. A wide class of minerals, for example, rutiles, ferrites, quartzites and so on belong to suitable materials for use as detoxifying medicaments, when reduced in size down to nanoparticles. These materials have heterocrystal structure, wherein lattice fragments are coupled by means of hydroxide ion (OH-). Preferably, these materials are subjected to a thermal destruction for purposes of production of nanoparticles, whereby a cooling mode is altered with a heating mode. In course of a cooling phase, the chemical bonds of the hydroxide ion undergo crystallization thereby destructing the lattice. The action of the alternating cooling/heating mode can be seen as internal blasts causing the matter to reduce its size down to nanometers, thereby yielding nanoparticles. Further details of the method of thermal destruction will be discussed with reference to Figure 1.

Next to their detoxifying action, as an additional effect, the nanoparticles of rutile, sphene, lopatire, perowskite, anatase, ilmenite, leukoxen, ferrite, quartzite exhibit anti-bacterial and anti-viral action and are suitable for use as a medicament with anti-viral or anti-bacterial action. They are applicable either for systemic administration, or for local application. Advantageously, the nanoparticles can be used for sanitary purposes, hygiene, including radiation protection and radiation hygiene. The anti-bacterial and anti-viral action of nanoparticles originating from rutile, sphene, lopatire, perowskite, anatase, ilmenite, leukoxen, ferrite and quartzite is based on their property to deactivate unsaturated chemical bonds of peptides, glycozydes and the like. Also, they can be used as coatings or applications on surgical threads, bandages. Alternatively, such nanoparticles can be used to coat intaersitial prothesis, like artificial cardiac valves, bypasses, etc. Therefore, such nanoparticles lend themselves for use as anti-bacterial and anti-viral material as such.

It is preferable to use minerals comprising dioxides of metals, like rutile, sphene, loparite, perowskite, anatase, ilmenite, lukoxen and ferrites for the source of nanoparticles, as the resulting nanoparticles can be used as a medicament to treat cancer due to their additional properties of a photosensitizer able to generate active oxygen, notably in singlet form when subjected to irradiation with light. The technical measure of the invention is based on the insight that nanoparticles of a dioxide of the biologically compatible metal have a plurality of advantageous effects when used as the photosensitizer. The nanoparticles have a maximum surface of contact, thereby improving the efficacy of the activation process, which is substantially a mass exchange process dependent on the area of reacting surfaces. Preferably, nanoparticles with dimensions of 0,5 - 200 nm are prepared, still preferably with dimensions of 0,5 - 100, still preferably with dimensions of 0,5 - 50 nm, because they are comparable in size with the molecule of glucose, still further increasing their chemical activity. The nanoparticles may have spheric and/or rod shapes, their dimensions being measured by per se known Atomic Force Microscope (AFH) or Stonnich Tunnel (ST). It is noted that the photosensitizer according to the invention may comprise various mixtures of nanoparticle sizes, whereby also relative percentage of nanoparticles of specific dimensions may vary. The term dimension relates to all dimensions of nanoparticles.

The nanoparticles of a metal-catalyst have a double action, first due to generic properties of nanoparticles, as indicated with reference to the foregoing, and, secondly, due to the properties of a photosensitizer, as they additionally catalyze a formation of active oxygen in response to irradiation with light, notably in a wide range of wavelengths, from ultra-violet to infrared range. Also, providing a photosensitizer in nanoparticle form has an additional advantageous effect of the matter being transparent to a wide range of wave lengths of light thereby improving an efficacy of the material response to light irradiation.

It is further noted that the photosensitizer comprising nanoparticles of titanium dioxide and iron dioxide, being a substantially transparent medium, can be activated by visible light. Therefore, such materials are perfectly suitable for use in a form of powder to be supplied locally on superficial targets. For example, a suitable amount of powder of nanoparticles comprising titanium-dioxide or iron dioxide can be applied to a target region and can be subjected to the ambient light, or, alternatively, it can be subjected to another activation, for example from a laser, beforehand or during a suitable clinical or cosmetic procedure. It is noted that for superficial application the active oxygen will be formed from the atmospheric oxygen and the action of the photosensitizer for catalyzing the production of active oxygen shall be as efficient. Active oxygen must be understood as oxygen with increased activation potential, notably (ε, Δ) singlet oxygen.

Alternatively, it is possible to prepare a suitably concentrated suspension from the nanoparticles prepared from rutile, sphene, loparite, perowskite, anatase, ilmenite, leukoxen or ferrite, which enables administration of the photosensitizer into a recipient systemically, intracavitary, or by means of injection. Preferred embodiments of a liquid suitable to be used in the invention comprise water, solution of a colloid, liquid comprising protein, for example albumin, or a physiologic salt solution. Preferably, the resulting suspension is prepared using a ratio of at least 0,5 mg of nanoparticles, notably titanium dioxide or iron dioxide per 1 liter of liquid. It is noted that minerals like rutile, sphene, loparite, perowskite, anatase, ilmenite, leukoxen and ferrite generally comprise a mass fraction of 10% - 30% of respective metal dioxides. Therefore, for those minerals the total mass of prepared nanoparticles should be selected at least 5 mg per 1 liter of liquid. It is further noted that when nanoparticles are being used as photosensitizer, they may act as catalysts for production of active oxygen forms, thereby not participating in chemical reactions. Therefore, the total amount of the photosensitizer is not a critical parameter for production of active oxygen, provided a minimum amount of 0,5 mg Ti02 or Fe02 or mixture thereof per liter of liquid is prepared. Because a surface interaction of the liquid with the ambient oxygen will not be sufficient to produce a necessary level of oxygenation of the suspension, oxygen can be supplied to the resulting suspension from a suitable vessel my means of a pump. Preferable partial pressure of oxygen within the suspension is about 40 - 100 mmHg, still preferably about 70 mm Hg. Preferably, the photosensitizer is activated by means of irradiation using a laser light with a wavelength in the range of 0,8 - 0,9 micrometer, which corresponds to a maximum absorption rate of oxygen and a region of optical transparency of tissues to light.

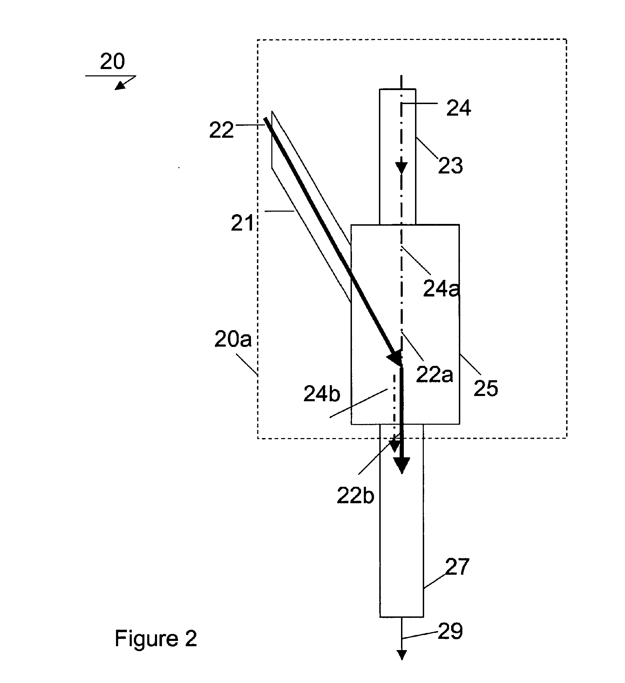

Still preferably, the photosensitizer is activated in a unit arranged to irradiate a suspension of the photosensitizer by a light source, notably a flow of the said suspension. By irradiating a flow of the suspension of the photosensitizer a production of active oxygen substantially in the whole volume of the suspension comprising photosensitizer is enabled. This step is advantageously carried out ex-vivo, for example as a preparatory step for photodynamic treatment of a suitable health disorder. Preferably, light intensity of at least 1 Joule/ml of the suspension comprising the photosensitizer is used. Still preferably, the light intensity is selected in the range of 2 -3 Joules/ml of the suspension of the photosensitizer. It is further preferable that the flow of the suspension comprising the photosensitizer is selected in the range of 1- 2 ml/second. Preferably, a suitable Y-shaped body is used for a unit, whereby a first arm of the Y-shaped body is used to supply a flow of the suspension of the photosensitizer into a main channel of the unit and a second arm of the Y-shaped body is used to supply light into the main channel of the Y-shaped body, preferably, by means of a suitably selected and attached optical fiber. Preferably, the flow rate of the suspension of the photosensitizer is selected in accordance with the light intensity supplied by the optical fiber. In general, a preferable relation between the flow rate and the light intensity lies in the range of 2-5 W of light energy per 1 ml of the suspension. Still preferably, the main channel of the Y-shaped body is attached to a storage cavity for storing the activated photosensitizer prior to its use. Preferably, the cavity is prepared in a sterile manner, so that the required volume of the activated photosensitizer can be extracted by, for example, an injection needle in a course of a medical or cosmetic treatment. A preferred embodiment of the unit will be described in more detail with reference to Figure 2. Alternatively, the unit can be arranged to conduct the activated photosensitizer to a supply unit which is used for administering the activated photosensitizer into the target region. In this case the flow of the photosensitizer advantageously serves as an optic conductor for the light waves, thereby further focusing a deposition of the active oxygen in the target region.

Still preferably, the suspension is ozonated, for this procedure per se known apparata for ozonating liquids can be used. The advantage of this step is the provision of an increased concentration of oxygen in the medium comprising the photosensitizer and a production of a variety of active oxygen types (e, Δ) in reaction to the activated photozensitizer. Preferably, the level of ozonation is kept within a range of 5 - 10 mg ozon per 1 liter of the suspension comprising the photosensitizer. The process of ozonation is advantageous, because it provides a variety of active oxygen forms in the suspension, like the singlet oxygen and ozone, which complement each other in their chemical and biological activity. Upon an interaction with other elements from environment the active oxygen and its secondary derivatives become a source of damage of a range of biologic objects which provide nutrition supply to cells. As a result, in the biological matter, subjected to an interaction with the compound according to the invention, fibrous processes are initiated, as well as processes of blood and lymphatic blockage, acting as mechanical barrier for blood and lymphatic circulation, thereby isolating, for example, a tumor from supply sources. As a result the metabolic activity and invasive properties of tumor cells are substantially reduced and the tumor cannot demonstrate its invasive and metastasic activity.

An alternative embodiment refers to the mineral comprising ferrite being reduced to nanoparticles and being used as a medicament for treating anemia's. Next to the advantages of the nanoparticles due to their specific chemical activity, presence of iron is used for treating of anemia's of various genesis, in particular of ferro-dificite origin. This technical measure is based on the insight that provision of iron inside cells positively influences mechanisms of protein synthesis, wherein iron-ferments play an active role. Due to the fact that nanoparticles are thousands of times smaller than the average size of a cell of a living organism, they can easily migrate inside the cell and participate in chemical reactions in a similar way to molecules of matter. Preferably, for injections a total dose of F202 is selected of about 10 mg, whereby for superficial application there is no limitation of the dose.

In a further alternative embodiment the mineral reduced to nanoparticles for use as a medicament comprises quartzite. Quarzites are characterized by presence of Si02, which in its nanoparticle form has an advantageous effect of initiating fibrous tissue. This advantageous effect, generally manifesting itself as a blood and lymphatic block, can be used for localization of cancerous tissue thereby for eliminating any source of nutrition of malignant cells. For administration of Si02 nanoparticles using injection a preferred dose of Si02 is about 10mg, for superficial applications there is no limitation. It is further noted that nanoparticles of Si02 can be used as transport agents, when chemically coupled to a suitable molecule. For example, nanoparticles of Si02 can be chemically coupled to silver oxides or dioxides, which can supplement the action of Si02 by its photosensitizing properties. Alternatively, a Si02-coupled Ag02 can be added to nanoparticles comprising dioxide of titanium or dioxide of iron thereby complementing their action as metal catalysts for production of active oxygen forms.

In a still further alternative embodiment, the nanoparticles of a heterocrystal mineral comprise a DNA-molecule are used as a medicament. Preferably, the nanoparticles are coupled to the DNA molecule, notably by electrostatic forces or by covalent bonds. It is noted that several embodiments of such coupling are envisaged, namely first, when a single nanoparticles is couples to a single DNA molecule, secondly, when a plurality of nanoparticles are coupled to a single DNA molecule, and third, when a plurality of DNA molecules are couples to a nanoparticle. This technical measure can be implemented by an introduction of, for example, sodium salt of a DNA-molecule, commercially known as a "Derinat", into a suspension comprising the said nanoparticles, preferably based on a proportion of 1 portion of 1,5% solution of sodium salt per 1 portion of nanoparticles comprising at least a dose of 0,5 mg of titanium dioxide, iron dioxide or silicon dioxide. When nanoparticles originating from Si02 are coupled to DNA molecule and, additionally to a suitable anti-metabolic agent, providing technical effect of improving deposition of the fibrogenic matter (Si02) inside a cell in once case, and further damaging the cell by action of a anti-metabolic agent, in the other case. When both DNA molecule and anti-metabolic agent are coupled to nanoparticles, a synergistic effect may occur between the two effects set forth in the foregoing, still further improving a medical action of nanoparticles on the recipient. This embodiment has a technical effect of still further increasing a selectivity of bio-chemical action of the said nanoparticles due to the fact that they are transported inside the cell by action of the DNA-molecule.

Still preferably, the nanoparticles, notably, of rutile, sphene, loparite, perowskite, anatase, ilmenite, leukoxen or ferrite, comprise an anti-metabolic agent are used as a medicament. This technical measure is based on the insight that the nanometric compound can be used as a transport agent to transport a further medicament inside a cell. The said coupling can be achieved by simply adding the anti-metabolic agent to the nanoparticles. A coupling of the anti-metabolic agent to the said nanoparticles provides a medicament with a cumulative cytotoxic action on the cell thereby still further improving an efficacy of the thus prepared substance. For example, the anti-metabolic agent may comprise a suitable chemotherapeutic material per se known in the art.

It is noted that although specific technical effects are explained with reference to isolated embodiments of nanoparticles produced by means of destruction of a heterocrystal mineral, various modifications of the embodiments, in particular, use of a mixture of disclosed minerals as a source of nanoparticles are contemplated as well. Nanoparticles for use as a medicament, notably nanoparticles comprising DNA molecule, and/or anti-metabolic agent are contemplated as well. It is further noted that production of the nanoparticles from the disclosed minerals provides a simple and economic way of effective production of medicaments as these minerals are present in nature in abundance. Some illustrations of the disclosed nanoparticles for use as medicaments will be discussed with reference to examples.

### Example 1: nanoparticles from rutile or ferrite for use as a medicament for treating cancer.

This field of application is based on the photosensitizing property of dioxides of metals, it being a production of active oxygen forms under irradiation of light. For a superficially located cancerous mass the activated photosensitizer in nanoparticle form is introduced superficially and around the mass, for example, using a suitable injection needle. The volume of the suspension comprising the activated nanoparticle photosensitizer must be at least several times greater than the volume of the mass. Preferably, it is 5 to 10 times greater than the volume of the mass. It is noted that it is possible to provide the injection needle with a Y-shaped body one arm being a supply channel for the suspension comprising the photosensitizer, the other arm being an optical channel supplying the light. Preferably, the light intensity is not less than 1 W/cm2. Still preferably, the light is supplied in the direction of the flow of the suspension and is subjected to a further spreading in the tissue conceived to be treated. When laser is selected for the light source and is allowed to propagate inside a tissue being subjected to photodynamic treatment, it has an additional effect of inducing local hyperthermia still further improving medical effect of the procedure. The flow rate of the liquid is preferably not less than 1 ml per 1 Joules of light energy. Preferably, to reduce pain sensation of the recipient a suitable anesthetic is introduced into the suspension prior to the procedure of intervention. Preferably, a 0.2% lidocaine and 10mg/l ozon are used.

### Case 1.

A patient B. aged 18, (dossier No. 212/01) was treated in the year 2000 for a recidive of a soft-tissue sarcoma in a right half of the chest. The histologically proven recidive appeared after a time of three months of a tumor resection. During examination a spherical node of about 4 cm diameter was detected in a region of post-operative scar, said node being static, firmly attached to a rib. No distant metastases were detected by means of ultrasonic and X-ray investigations. The patient received interstitially 200 ml of the suspension comprising 1 mg of nanoparticles prepared by thermal destruction of rutile, irradiated by a laser light of 0,88 micrometer wavelength with an intensity of 5 W during 4 minutes. The procedure was repeated twice with a weekly interval. Tissue temperature in the course of treatment was not elevated by more than 0,8 degrees Celsius. A biopsy conducted three days after the second procedure demonstrated that the tumor belonged to sacroma's. After one year of follow-up no tumor growth was detected and in place of the recidive of 4cm in diameter one finds 1 cm node of fibrous tissue. During subsequent follow-up procedures no tumor manifestation was detected.

### Case 2.

For deep seated tumors which cannot be localized by a mere inspection, diagnostic means are used for presenting data on the spatial location of the target region comprising the tumor and the dimensions thereof. Also in this embodiment, a suitably arranged injection needle can be used for depositing the activated photosensitizer in the tumor and/or around it. A patient K, aged 64 (dossier No. 4322) in 2001 was hospitalized in an urologic clinic due to disurrhea. In the course of examination adenocarcinoma of an upper portion of the left lobe of prostate gland was detected. Adjuvant to catheterization of the bladder, a treatment by application of nanoparticles of rutile origin was carried out. Both lobes of the prostate gland received 250 ml of suspension comprising 1,5 mg dioxide of titanium in a solution of a silver dioxide irradiated by a laser light of 0.84 micrometer wavelength, intensity of 6 W, during 8 minutes. The tissue temperature was controlled and did not elevate by more than 1,2 degree Celsius in the course of treatment. The phenomenon of disurrhea has terminated and diagnostic screening showed solely a fibrous tissue in place of original tumor. This state was confirmed by a further follow-up three years after the treatment according to the invention.

### Case 3.

A female patient L., age 59 (dossier No. 176/32) was included in a group of volunteer patients to receive treatment by means of administration of nanoparticles originating from the mineral according to the invention. The patient was diagnosed with a ductal type carcinoma of the left mamma, staged T3N2Mx. The radiotherapy received by the patient was not successful. During her investigation prior to treatment in accordance with the invention a tumor mass of substantial dimensions (10 cm3) was detected, which also had developed an ulcer of 30 cm2 area. In the course of treatment the patient received 10 times intro-paratumoural injections with activated photosensitizer comprising 20% titanium dioxide, 30 % of calcium dioxide, and at least 15% of silicon dioxide. 5 mg of the photosensitizer was used to produce a suspension of about 1 liter in volume using sterile water, whereto 5 ml of 1.5% Derinat was added and 5 mg of the anti-metabolic agent in the form of doxorubicin. Next to the intra- and para-tumoral injections the lymph nodes received the suspension of the activated photosensitizer as well. During a course of the follow-up no manifestation of cancerous cells in the treated volume was detected, which was confirmed by substantial hystologic analysis. The area of the ulcer defect was reduced by about 5 times and no signs of any cancerous process can be seen as well. dermal defect of ulcer was closed by plastic surgery.

### Example 2: use of nanoparticles prepared from heterostructure material for use as a medicament for wound healing.

### Case 4.

A female patient 76 years (case No. 318/A), after being a patient of a plurality of oncology clinics during a period of more than four years due to presence of a substantial (18 cm3) cancerous ulcer on her left check. Conventional attempts to close the dermal defect were not successful. Then she was subjected to a monthly course of treatment with nanoparticles prepared using a mixture of ferrite and quartzite, whereby the nanoparticles were administered in form of dry powder. Upon the application of the powder on the ulcer, it was irradiated with a halogen lamp with intensity of 3 W/cm2 in dose of 1,3 kJoule. After the treatment the skin defect disappeared, whereby the prints of ulcer showed forms of fibrous cells in phases of apoptosis. It is noted that although the present example shows a cumulative action of nanoparticles from ferrite and quartzite, the former was used to treat cancer and the latter was used as an adjuvant therapeutic agent for stimulating fibrosis to close the skin defect. It must be understood, that clinical use of nanoparticles prepared from quartzite for wound healing is contemplated hereby as such.

### Example 3: nanoparticles from heterostructure mineral (ferrite) as a medicament for treating anemia.

### Case 5.

A male patient, 31 years (case No. 1272/A) was operated in 2003 for melanoma of left half of his back, subjected to adjuvant chemotherapy and radiotherapy and therefore manifested acute anemia (hemoglobin level of 6,8 g/liter). He was treated with nanoparticles prepared from ferrite, the treatment was set forth with a background of non-healing wound at a place of surgical intervention. The treatment comprised 10 session of administration of 10mg of nanoparticles (22% mass fraction of iron dioxide) prepared in a total volume of 150 ml aqueous suspension. During injection of the thus prepared suspension, it was irradiated by a laser light with wavelength of 0,88 micrometer with a total dose of 1,4 kJoule. Already after the third injection the hemoglobin concentration in blood was increased by 50 % up to the level of 9 mg/l and the wound showed granulation process leading to total wound healing.

These and other aspects of the invention will be discussed in further details with reference to Figures.
Figure 1 shows in a schematic way an embodiment of an apparatus for production of nanoparticles using a thermal destruction method.
Figure 2 shows in a schematic way an embodiment of an Y-shaped body for activation of nanoparticles used as photosensitizer.

Figure 1 presents a schematic illustration of an embodiment of an apparatus 10 for production of nanoparticles using thermal destruction method. For this purpose a layer 12 of a heterocrystal mineral is provided on a suitable carrier 12a for enabling thermal contact with a suitable source 14a, 14b, 14c, 14d. Preferably, a thickness of the layer 12 in z-direction, schematically shown in Figure 1, is in the order of several millimeters, still preferably it is selected in the order of one millimeter, corresponding to a size of the lattice of rutiles, shpenes, loparites, anatases, ilmenites, leukoxenes, ferrites, quartzites. The dimension of the layer in x-direction, schematically shown in Figure 1, can be as large as one meter. Preferably, ceramics is selected for the carrier 12a, which can conduct energy in the range of 9 - 15 W/cm2. The sources 14a, 14b, 14c, 14d comprise thermo-elements, electrically coupled to a power supply source 16. Preferably, the power supply source is controlled by a processor 18, which is preferably operated by a suitable computer program 18a. Still preferably, the power supply unit 16 and the control unit 18 are implemented as an integral device 17. In accordance with the method of the invention, the power supply source produces pulses of constant current with amplitude of at least 10 A/mm2, which are transferred to the thermo-elements 14a, 14b, 14c, 14d. A thermo-element, conceived to operate in accordance with a per se known thermoelectric effect of Peltier-Zeebeck may be used in the method according to the invention, whereby upon application of a current of a suitable polarity the solder joints of the thermo-element act as coolers or as heaters. It is noted that application of a single thermo-element or a plurality of thermo-elements is contemplated. In an embodiment of the method according to the invention a pulse of a direct polarity is applied during, for example a period of 10⁻⁴ to 1 second, as a result of which temperature of the surface of thermo-element facing the layer 12 instantly decreases down to at least - 50 degrees Celcius, preferably to -73 degrees Celcius. This causes the material of the layer 12 to substantially cool, whereby the hydroxide groups of water molecules crystallize thereby induce miniature blasts in the material of the layer 12. After this, the pulse of the direct polarity is changed to a pulse of an inverse polarity, thereby causing the surface of thermo-elements facing the layer 12 instantly to increase its temperature at least to + 80 degrees Celsius, preferably to +95 degrees Celsius, thereby melting water component in the crystal and disrupting lattice structure due to thermal vibration of lattice. After this, the polarity of the power supply unit 16 is altered again, causing the crystallization of water component. Preferably, such alteration is repeated at least for several times, for a period of, for example 1 sec to 1 minute. After a repetition of the cooling and heating modes, the layer 12 is destructed down to nanoparticles. A number of the repetitions can be selected in accordance with desired dimension of nanoparticles. In general, in accordance with the method of the invention nanoparticles with dimensions of 0,5-200 nm can be produced. It is also possible to produce a mixture of nanoparticles having various dimensions in the range of 0,5 - 200 nm. It is further possible to vary relative percentages of nanoparticles with specific dimensions in the mixture.

Figure 2 presents in a schematic way an embodiment 20 of a unit for activating a flow of the photosensitizer. The mixing unit 20a comprises a Y-shaped body, one arm 21 being conceived to receive, notably, a flow of the suspension of the photosensitizer 21, the other arm 23 being conceived to supply light waves 24 from a light source (not shown), preferably using a suitable optical fiber. The Y-shaped body of the unit 20a is arranged in such a way that the liquid photosensitizer 22a is fully irradiated by the light beam 24a. This arrangement ensures complete activation of the photosensitizer in the whole volume being irradiated. The portion 25 of the Y-shaped body may be manufactured with considerable dimensions, to contain the whole volume of the photosensitizer conceived to be used during a treatment procedure. It is found to be sufficient to provide the portion 25 with a 10 - 500 ml volume. Preferably, the volume of the portion 25 is selected around 50 ml. When the photosensitizer is activated and is stored in the compartment 25, a supply unit 27 can be connected to its distal portion. The supply unit may comprise a catheter, an injection needle, or a spraying device used for administering the photosensitizer 29 to the target region of the recipient. In case when the injection needle, or a catheter are used it may be preferable not to interrupt the flow 22b of the activated photosensitizer allowing it to conduct the optical radiation into the tissues of the recipient. In this way the administration of the activated photosensitizer is performed substantially in real-time with its activation, whereby the liquid photosensitizer acts as an optical conductor of the light beam into the tissue. This procedure has an advantageous clinical effect as the nanometric photosensitizer instantly deposits the dose of the activated oxygen and the optical dose at the micro-level as well. In case when the laser light is used the optical dose thus delivered causes additional tissue damage due to local ablation due to laser-induced hyperthermia. Preferably, the laser light with the wavelength of 0,8 - 0,9 micrometer is used, corresponding to the maximum absorption rate of oxygen and the region of optical transparency of tissue.

## Claims

1. Nanoparticles of a heterocrystal mineral for use as a medicament.

2. Nanoparticles according to Claim 1, wherein the mineral comprises at least one of rutile, sphene, loparite, perowskite, anatase, ilmenite, leukoxen, ferrite, quartzite.

3. Nanoparticles according to any one of the preceding Claims 1 or 2, wherein nanoparticles comprise a DNA molecule.

4. Nanoparticles according to Claims 1-3, wherein the nanoparticles comprise an anti-metabolic agent.

5. A composition comprising nanoparticles as defined in any one of the preceding Claims 1-4 and a fluidum, preferably water or air.

6. Use of nanoparticles of a heterocrystal mineral according to any one of the preceding Claims 1-4 for manufacturing a medicament for treatment of a bacterial or a viral infection.

7. Use of nanoparticles of a heterocrystal mineral according to any one of the preceding Claims 1- 4 for manufacturing a medicament for treatment of cancer.

8. Use of nanoparticles of a hetercrystal mineral according to Claims 1- 4 for manufacturing a medicament for healing a wound.

9. A method of producing nanoparticles from a heterocrystal mineral, said method comprising subjecting a layer of the mineral to a repetitive application of cooling and heating.

10. A method according to Claim 9, wherein for the step of repetitively applying the cooling and the heating a thermo-element is used.
